Europäisches Patentamt

European Patent Office

Office européen des brevets

(19)

(11) EP 1 566 839 A1

(12) EUROPEAN PATENT APPLICATION

(43) Date of publication:
24.08.2005 Bulletin 2005/34

(51) Int Cl.7: H01L 27/00

(21) Application number: 05075208.8

(22) Date of filing: 27.01.2005

(84) Designated Contracting States:
AT BE BG CH CY CZ DE DK EE ES FI FR GB GR
HU IE IS IT LI LT LU MC NL PL PT RO SE SI SK TR
Designated Extension States:
AL BA HR LV MK YU

(30) Priority: 27.01.2004 KR 2004005154

(71) Applicant: Samsung SDI Co., Ltd.
Gyeonggi-do (KR)

(72) Inventors:
• Kim, Mu-Hyun
  Suwon-si gyeonggi-do (KR)
• Kim, Kyong-Do
  Suwon-si Gyeonggi-do (KR)

(74) Representative: Hengelhaupt, Jürgen et al
Anwaltskanzlei
Gulde Hengelhaupt Ziebig & Schneider
Wallstrasse 58/59
10179 Berlin (DE)

(54) **Organic light emitting display and method of fabricating the same**

(57) An organic light emitting display and method of fabricating the same are provided, in which surface corner portions of contact holes and via holes are formed to be curved so that short-circuit failures may be reduced. The organic light emitting display includes: a semiconductor layer (211) formed on a non-emission region (210) of an insulating substrate (200), and having source (213) and drain (214) regions; a thin film transistor formed on the substrate, and having source (215) and drain (216) electrodes electrically connected to the semiconductor layer through a connection hole (261,262); a bottom electrode (231) formed on an emission region (230) of the insulating substrate, and connected to one electrode of the source and drain electrodes through a connection hole (271); an organic emission layer (232) formed on the emission region of the bottom electrode; and a top electrode (233) formed on the organic emission layer, wherein the connection hole has a curved surface corner portion.

EP 1 566 839 A1

## Description

## CROSS-REFERENCE TO RELATED APPLICATION

This application claims priority to and the benefit of Korean Patent Application No. 2004-5154, filed January 27, 2004, the disclosure of which is incorporated by reference in its entirety.

## BACKGROUND OF THE INVENTION

1. Field of the Invention

The present invention relates to an organic light emitting display and method of fabricating the same and, more particularly, to an organic light emitting display and method of fabricating the same in which surface corners of a connection hole are formed to be curved which is formed between upper and lower conductive layers to interconnect the upper conductive layer and the lower conductive layer so that short-circuit failures may be reduced.

2. Description of the Related Art

In recent years, development for an active matrix organic light emitting display (AMOLED) as an active matrix flat panel display using an organic light emitting (EL) diode is being progressed, which is applied to a cellular phone or the like, so that thickness and size thereof are remarkably reduced, manufacturing cost is reduced and a process thereof is also simplified.

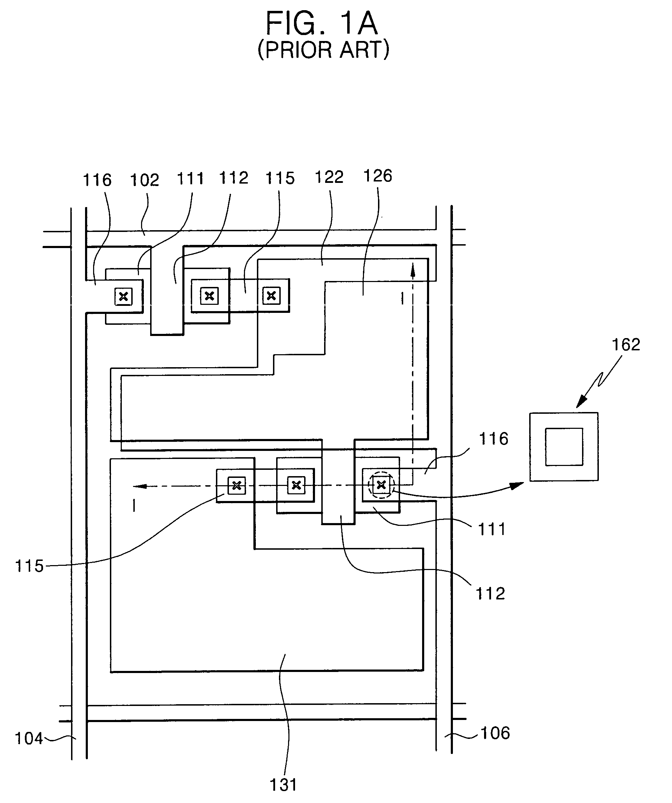

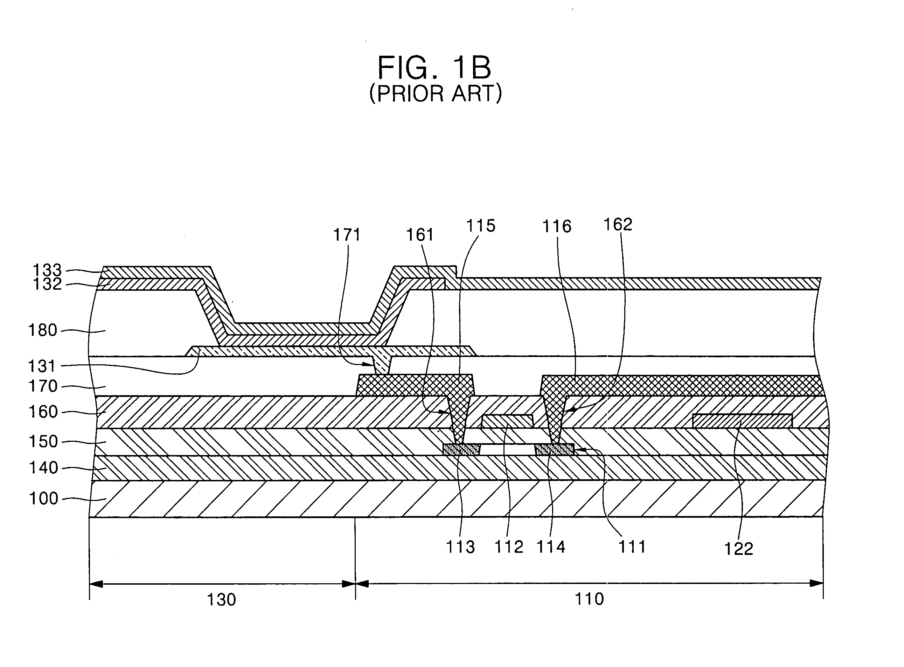

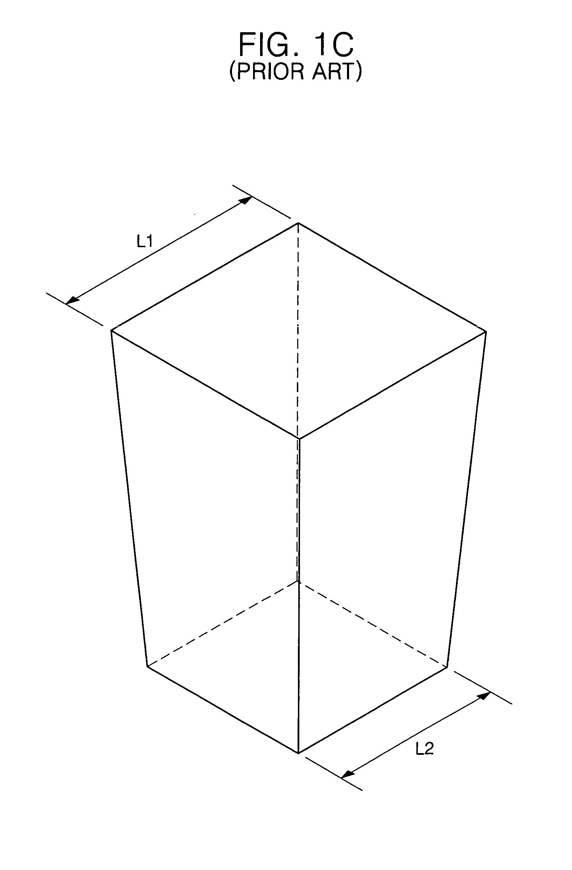

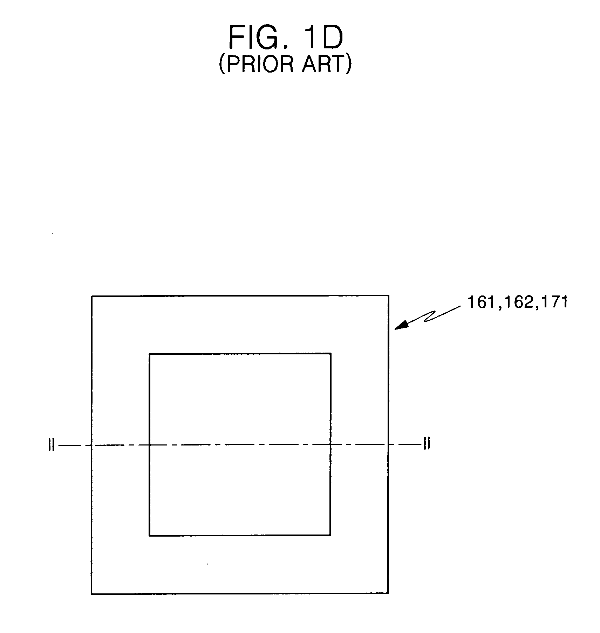

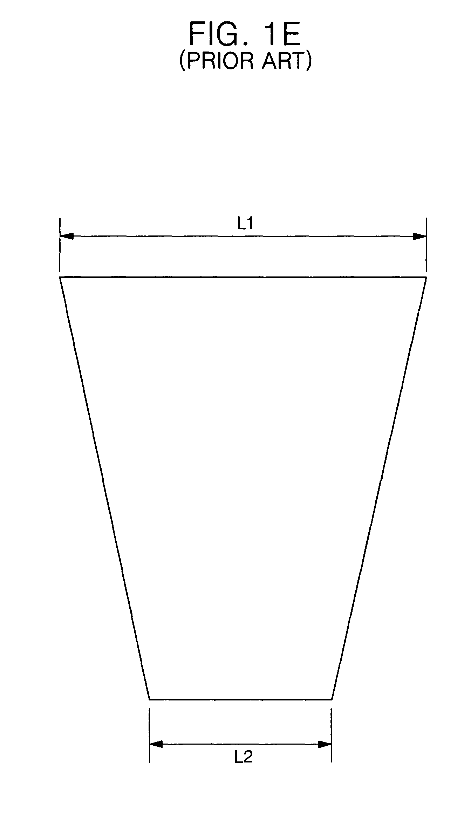

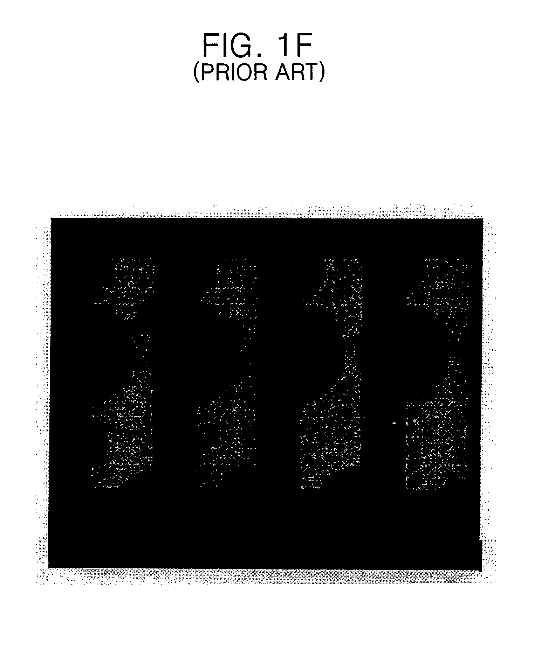

FIG. 1A is a plan view of a conventional organic light emitting display. The organic light emitting display of FIG. 1A is illustrated to consist of two transistors and one capacitor.

FIG. 1B is a cross-sectional view taken along the line I-I of FIG. 1A. Referring to FIG. 1B, the conventional organic light emitting display has an emission region 130 where a lower electrode 131 as a pixel electrode, an organic emission layer 132 and a upper electrode 133 are formed, and a non-emission region 110 where two thin film transistors (TFT) and a capacitor are formed.

In the non-emission region 110, a buffer layer 140 is formed on a transparent insulating substrate 100 such as a glass substrate, and amorphous silicon is deposited on the buffer layer 140, and then patterned and crystallized to form a semiconductor layer 111. A gate insulating layer 150 is then formed on the entire surface of the substrate, and a metal material for forming a gate electrode is deposited on the gate insulating layer 150 and then patterned to form a gate 112 on the semiconductor layer 111, while a capacitor bottom electrode 122 is formed. In this case, a gate line 102 shown in FIG. 1A is simultaneously formed while the gate 112 and the capacitor lower electrode 122 are formed.

Subsequently, impurities having a predetermined conductivity type such as a P type or an N type are ion-implanted into the semiconductor layer 111 to form source and drain regions 113 and 114.

An inter-insulating layer 160 is then formed on the entire surface of the substrate, and the inter-insulating layer 160 and the gate insulating layer 150 are etched so as to expose portions of the source and drain regions 113 and 114 to thereby form contact holes 161 and 162 for source and drain electrodes.

Subsequently, a metal material for forming the source and drain electrodes is deposited on the inter-insulating layer 160 to form the source and drain electrodes 115 and 116 which are in contact with the source and drain regions 113 and 114 through the contact holes 161 and 162, respectively. In this case, a capacitor top electrode 126 which extends from one electrode of the source and drain electrodes 115 and 116, for example, the drain electrode 116, is formed while a data line 104 and a power supply line 106 shown in FIG. 1A are formed.

A passivation layer 170 is then formed on the inter-insulating layer 160. The passivation layer 170 is etched so as to expose a portion of the other electrode of the source and drain electrodes 115 and 116, for example, the source electrode 115 to thereby form a contact hole 171 for a pixel electrode.

A transparent conductive layer is deposited on the passivation layer 170 of the emission region 130 and then patterned to form a bottom electrode 131 which is in contact with the source electrode 115 through the contact hole 171 for the pixel electrode.

An insulating layer 180 for planarization is formed on the passivation layer 170, and an opening 181 is formed so as to expose the lower electrode 131. An organic emission layer 132 is formed on the planarization layer 180 including the opening 181, and a top electrode 133 is formed thereon.

When a taper angle of the contact hole of the conventional organic light emitting display having the above-described configuration is large, pin-hole failures occurred at a stepped portion near the contact hole.

To cope with such a problem, taper angles at edge portions of the contact holes 161, 162, and 171 are alleviated to prevent the failures from occurring.

A process of forming the contact hole of the conventional organic light emitting display in which the taper angle is alleviated will be described with reference to FIGs. 1C to 1F.

FIG. 1C is a perspective view illustrating a shape of the contact holes 161, 162, and 171, FIG. 1D is a plan view of the contact holes 161, 162, and 171, and FIG. 1E is a cross-sectional view taken along the line II-II of FIG. 1C.

Referring to FIGs. 1C to 1E, corners of top and bottom surfaces of each of the contact holes 161, 162, and 171 are angled, and the length L1 of the top surface of each of the contact holes 161, 162, and 171 is formed to be larger than the length L2 of the bottom surface of each of the contact holes 161, 162, and 171.

That is, the contact holes 161, 162, and 171 are formed to have inclined sides so as to have taper angles.

The pin-hole failures may be reduced to some extent by means of the alleviated taper angle, however, a short-circuit failure may occur due to an edge open at the angled corner on the surface in the conventional organic light emitting display.

FIG. 1F is an enlarged view of an emission region when the short-circuit failure occurred, wherein problematic dark spots may occur at the corners of the contact holes 161, 162, and 171 when the short-circuit failures occur.

## SUMMARY OF THE INVENTION

The present invention, therefore, solves aforementioned problems associated with conventional devices by providing an organic light emitting display and method of fabricating the same in which surface corner portions of contact holes and via holes are formed to be curved to reduce the short-circuit failures.

The present invention also provides an organic light emitting display allowing durability to be lengthened and image quality to be enhanced.

In an exemplary embodiment of the present invention, an organic light emitting display includes: a semiconductor layer formed on a substrate, and having source and drain regions; a thin film transistor formed on the substrate, and having source and drain electrodes electrically connected to the semiconductor layer through a connection hole; a lower electrode formed on an emission region of the substrate, and connected to one electrode of the source and drain electrodes through a connection hole; an organic emission layer formed on the emission region of the lower electrode; and a upper electrode formed on the organic emission layer, wherein of the connection hole has a curved surface corner portion.

A corner of a bottom surface of the connection hole may have a curvature; a center of the curvature may be present on the bottom surface; a top surface of the connection hole may have a point which is positioned on the same axis vertically extending from a surface including a center of a radius of curvature of the bottom surface, as a center of a radius of curvature; the radius of curvature of the top surface of the connection hole may be larger than that of the bottom surface; and all of the curvature of the top surface and the curvature of the bottom surface of the connection hole may be formed at respective four corners.

Corners of the top and bottom surfaces of the connection hole may be circular; a point positioned on the same axis vertically extending from a surface including the center of the radius of the top surface may be the center of the radius of the bottom surface; the radius of the top surface of the connection hole may be larger than that of the bottom surface; a connection hole formed on the insulating layer to insulate the source and drain regions from the source and drain electrodes may serve as a contact hole; and a connection hole formed on the insulating layer to insulate the source and drain electrodes from the lower electrode may serve as a via hole. The lower electrode may be an anode electrode.

In another exemplary embodiment according to the present invention, a method of fabricating an organic light emitting display includes: forming a semiconductor layer having source and drain regions on a substrate; forming a thin film transistor provided with source and drain electrodes electrically connected to the semiconductor layer through a connection hole on the substrate; forming a lower electrode connected to one electrode of the source and drain electrodes through a connection hole on an emission region of the substrate; forming an organic emission layer on the emission region of the lower electrode; and forming a upper electrode on the organic emission layer, wherein the connection hole has a curved surface corner portion.

A corner of a bottom surface of the connection hole may have a curvature; a center of the curvature may be is present on the bottom surface; a top surface of the connection hole may have a point which is positioned on the same axis vertically extending from a surface including a center of a radius of curvature of the bottom surface, as a center of a radius of curvature; the radius of curvature of the top surface of the connection hole may be formed to be larger than that of the bottom surface; and all of the curvature of the top surface and the curvature of the bottom surface of the connection hole may be formed at respective four corners.

Corners of the top and bottom surfaces of the connection hole may be formed to be circular; a point positioned on the same axis vertically extending from a surface including the center of the radius of the top surface may be the center of the radius of the bottom surface; and the radius of the top surface of the connection hole may be formed to be larger than that of the bottom surface.

## BRIEF DESCRIPTION OF THE DRAWINGS

The above and other features of the present invention will be described in reference to certain exemplary embodiments thereof with reference to the attached drawings in which:

FIG. 1A is a plan view of a conventional organic light emitting display;
FIG. 1B is a cross-sectional view of the conventional organic light emitting display taken along the line I-I of FIG. 1A;
FIG. 1C is a perspective view illustrating a shape of a contact or via hole shown in FIG. 1A;
FIG. 1D is a plan view of the contact or via hole shown in FIG. 1C;

FIG. 1E is a cross-sectional view of the contact or via hole taken along the line II-II of FIG. 1 D;

FIG. 1 F is an enlarged view of an emission region illustrating a short-circuit failure;

FIG. 2A is a plan view of an organic light emitting display in accordance with a first embodiment of the present invention;

FIG. 2B is a cross-sectional view of an organic light emitting display taken along the line III-III of FIG. 2A in accordance with the first embodiment of the present invention;

FIG. 2C is a perspective view illustrating a shape of a contact or via hole shown in FIG. 2A;

FIG. 2D is a plan view of the contact or via hole shown in FIG. 2C;

FIG. 2E is a cross-sectional view of the contact or via hole taken along the line IV-IV of FIG. 2D;

FIG. 3A is a plan view of an organic light emitting display in accordance with a second embodiment of the present invention;

FIG. 3B is a perspective view illustrating a shape of a contact or via hole shown in FIG. 3A;

FIG. 3C is a plan view of the contact or via hole shown in FIG. 3A; and

FIG. 3D is a cross-sectional view of the contact or via hole taken along the line VI-VI of FIG. 3C.

## DETAILED DESCRIPTION OF THE INVENTION

The present invention will now be described more fully hereinafter with reference to the accompanying drawings, in which preferred embodiments of the invention are shown.

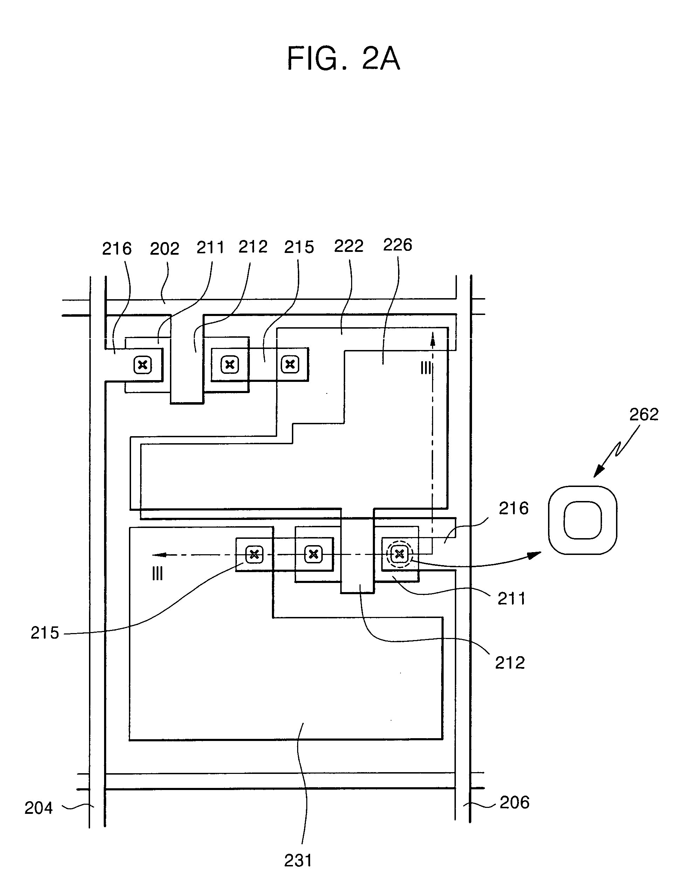

FIG. 2A is a plan view of an organic light emitting display in accordance with a first embodiment of the present invention.

The organic light emitting display of FIG. 2A is illustrated to have two transistors and one capacitor.

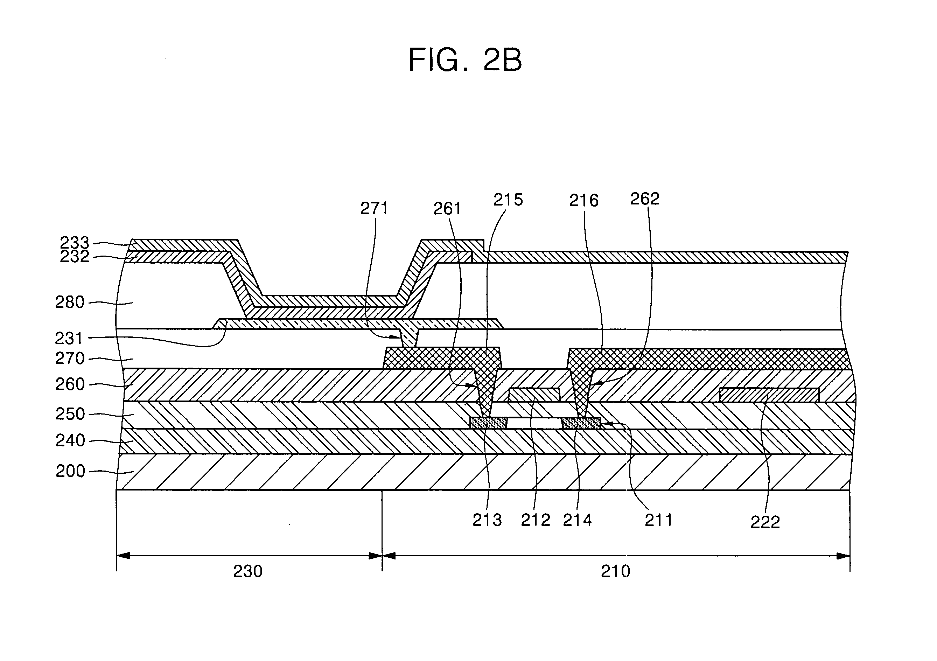

FIG. 2B is a cross-sectional view of an organic light emitting display taken along the line III-III of FIG. 2A.

Referring to FIG. 2B, in a non-emission region 210 of the organic light emitting display according to the first embodiment of the present invention, a buffer layer 240 is formed on a transparent substrate 200 such as a glass substrate, and amorphous silicon is deposited on the buffer layer 240, which is then patterned and crystallized to form a semiconductor layer 211. A gate insulating layer 250 is then formed on the entire surface of the substrate, and a metal material for forming a gate electrode is deposited on the gate insulating layer 250 and then patterned to form a gate 212 on the semiconductor layer 211, while a capacitor bottom electrode 222 is formed. In this case, a gate line 202 shown in FIG. 2A is simultaneously formed while the gate 212 and the capacitor bottom electrode 222 are formed.

Subsequently, impurities having a predetermined conductivity type such as a P type or an N type are ion-implanted into the semiconductor layer 211 so that source and drain regions 213 and 214 are formed.

An inter-insulating layer 260 is then formed on the entire surface of the substrate, and the inter-insulating layer 260 and the gate insulating layer 250 are etched so as to expose some portions of the source and drain regions 213 and 214 to thereby form first and second connection holes 261 and 262 for source and drain electrodes.

Subsequently, a metal material for forming source and drain electrodes is deposited on the inter-insulating layer 260 to form the source and drain electrodes 215 and 216 which are in contact with the source and drain regions 213 and 214 through the first and second connection holes 261 and 262, respectively. In this case, a capacitor upper electrode 226 which extends from one electrode of the source and drain electrodes 215 and 216, for example, the drain electrode 216, is formed while a data line 204 and a power supply line 206 shown in FIG. 2A are formed.

A passivation layer 270 is then formed on the inter-insulating layer 260. The passivation layer 270 is etched to expose a portion of the other electrode of the source and drain electrodes 215 and 216, for example, the source electrode 215 to thereby form a third connection hole 271.

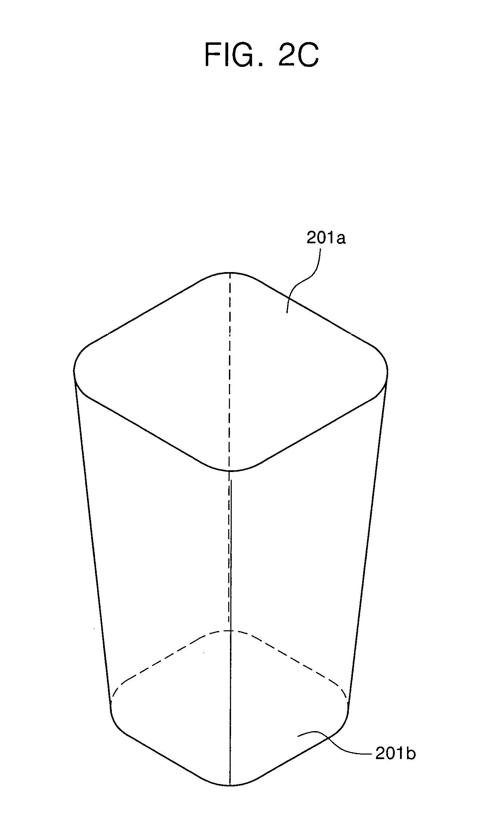

In this case, a process of forming the first to third connection holes shown in FIG. 2A will be described with reference to FIGs. 2C to 2E.

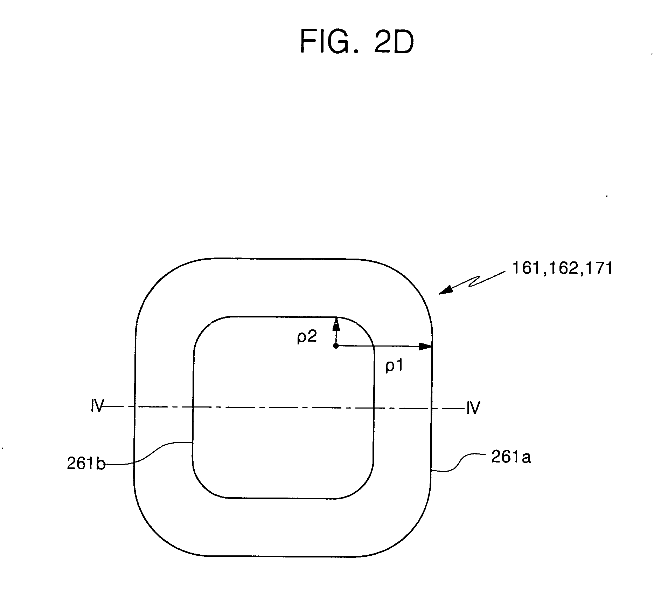

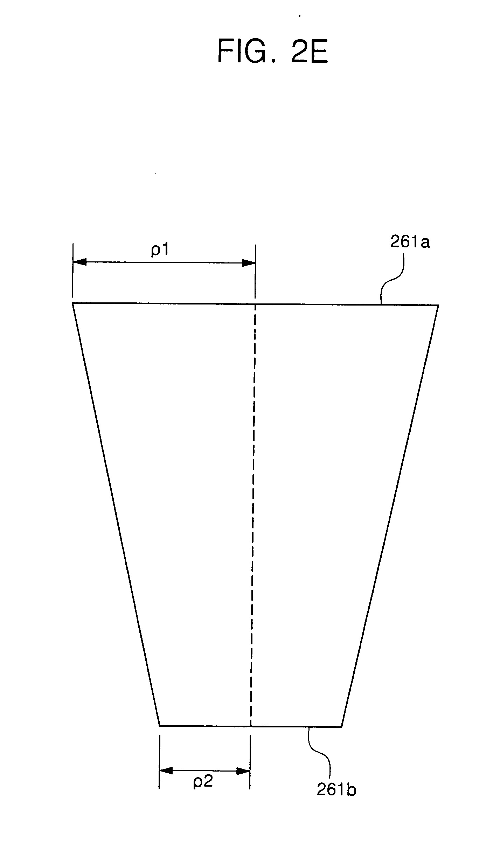

FIG. 2C is a perspective view illustrating a whole shape of each of the first to third connection holes 261, 262, and 271, FIG. 2D is a plan view of the first to third connection holes 261, 262, and 271, and FIG. 2E is a cross-sectional view taken along the line IV-IV of FIG. 2D.

When the first to third connection holes 261, 262, and 271 are formed, four corners of the planar top surface 261a are curved and four corners of the bottom surface 261b are curved as shown in FIG. 2D, and the first to third connection holes 261, 262, and 261 are formed to be tapered as shown in FIG. 2E, so that the top surface 261 a is formed to be wider than the bottom surface 261b. This may be expressed as the equation below:

$$\rho 1 > \rho 2 > 0$$

where, $\rho 1$ indicates a radius of curvature of the top surface 261a of the first to third connection holes 261, 262, and 271, and $\rho 2$ indicates a radius of curvature of the bottom surface 261b of the first to third connection holes 261, 262, and 271.

Each of four corners of the bottom surface 261 b of the first to third connection holes 261, 262, and 271 has a curvature, and a center of the radius of curvature of the bottom surface 261 b is present on the bottom surface 261 b, and the top surface 261 a of the first to third connection holes 261, 262, and 271 has a center of the radius of curvature (ρ 1) which is positioned on the same axis vertically extending from the surface including the center of the radius of curvature (ρ 2) of the bottom surface 261b, and the radius of curvature (ρ 1) of the top surface 261 a of the first to third connection holes 261, 262, and 271 is larger than the radius of curvature (ρ 2) of the bottom surface 261b, and not only four corners of the bottom surface 261b of the first to third connection holes 261, 262, and 271 but also four corners of the top surface 261 a are curved, so that a short-circuit failure caused by the edge open of the four corners may be prevented.

A transparent conductive layer is then deposited on the passivation layer 270 of the emission region 230 and then patterned to form a lower electrode 231 which is in contact with the source electrode 215 through the third connection hole 271.

An insulating layer 280 for planarization is formed on the passivation layer 270, and an opening 281 is formed to expose the lower electrode 231. An organic emission layer 232 is formed on the planarization layer 280 including the opening 281, and a upper electrode 233 is formed thereon.

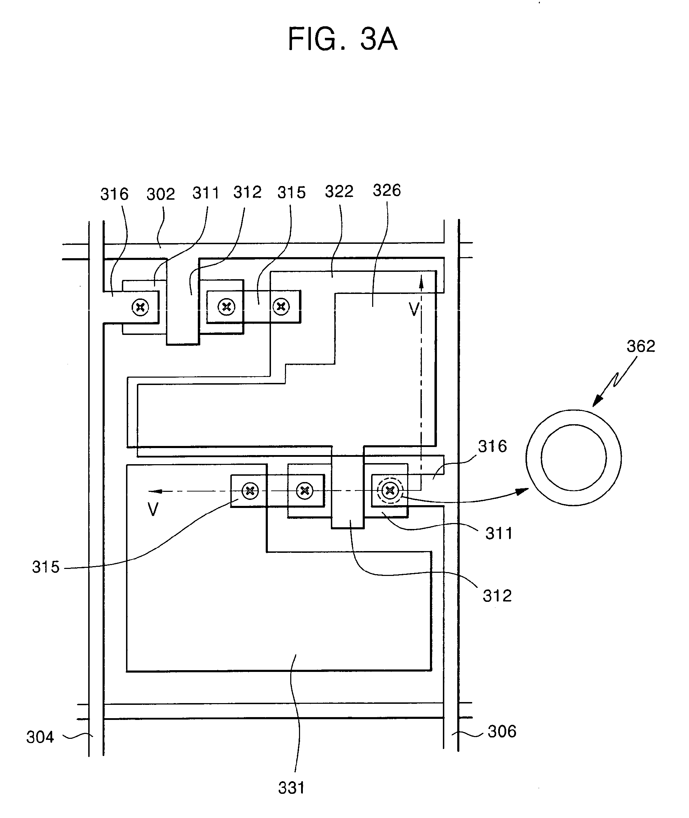

FIG. 3A is a plan view of an organic light emitting display in accordance with a second embodiment of the present invention.

The organic light emitting display of FIG. 3A is illustrated to have two transistors and one capacitor.

A cross-sectional structure taken along the line V-V of FIG. 3A has the same cross-sectional structure as FIG. 2B, and a fabrication method according to the second embodiment may be readily understood by those skilled in the art, so that a description thereof will be omitted.

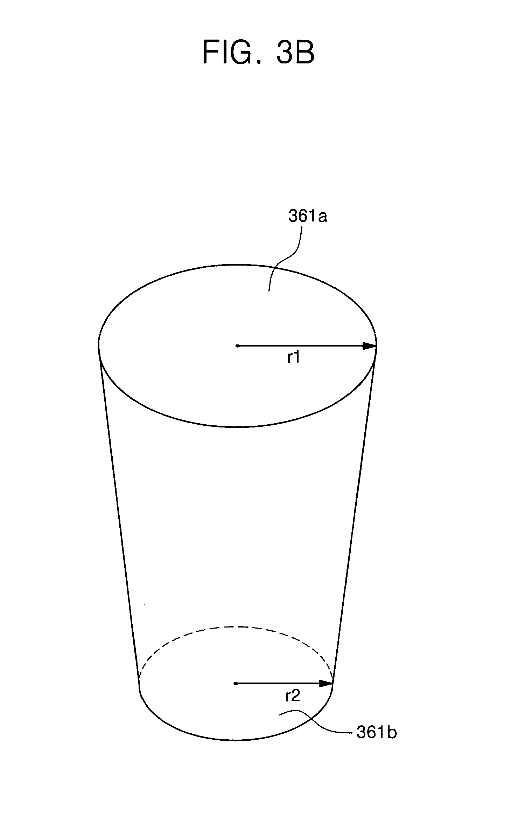

However, a process of forming shapes of the first to third connection holes 261, 262, and 271 shown in FIG. 4A will be described with reference to FIGs. 3B to 3D in the second embodiment.

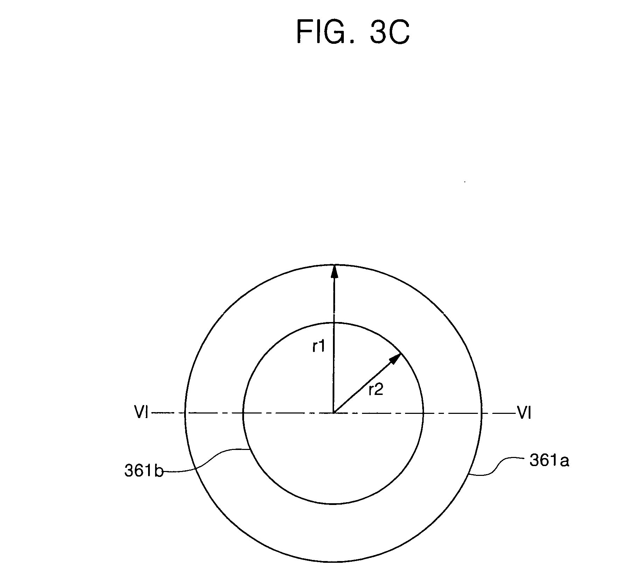

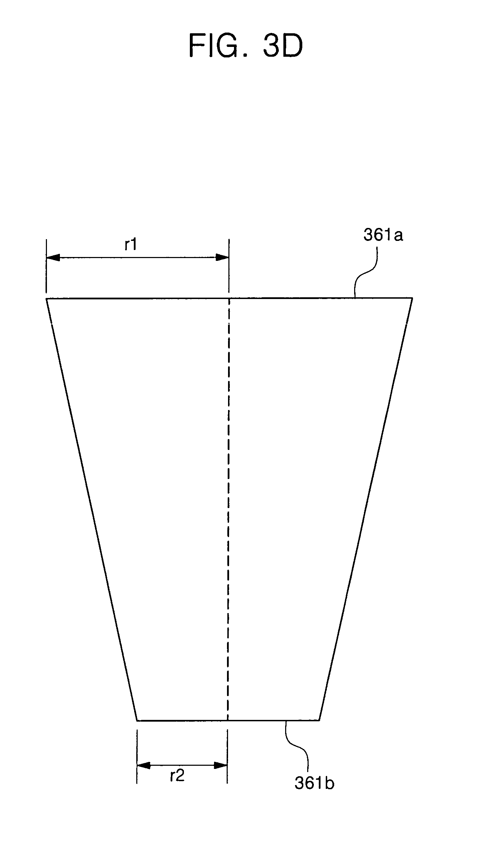

FIG. 3B is a perspective view illustrating a whole shape of each of the first to third connection holes 261, 262, and 271, FIG. 3C is a plan view of the connection holes 261, 262, and 271, and FIG. 3D is a cross-sectional view taken along the line VI-VI of FIG. 3C.

When the first to third connection holes 261, 262, and 271 are formed, top and bottom surface are circular as shown in FIG. 3C, and the first to third connection holes 261, 262, and 271 are formed to be tapered so that the top surface is wider than the bottom surface as shown in FIG. 3D.

This may be expressed as the equation below:

$$r1 > r2 > 0$$

where r1 indicates a radius of the top surface of each of the first to third connection holes 261, 262, and 271, and r2 indicates a radius of the bottom surface of each of the first to third connection holes 261, 262, and 271.

Corners of the top and bottom surfaces of the first to third connection holes 261, 262, and 271 are circular, and the bottom surface has a point which is positioned on the same axis vertically extending from the surface including the radius center of the top surface, as a radius center. The radius of the top surface of the connection hole is formed to be larger than that of the bottom surface, so that a short-circuit failure caused by the edge open of the corners of the first to third connection holes 261, 262, and 271 may be prevented.

Connection holes which are formed on the inter-insulating layer and the gate insulating layer for insulating the source and drain regions from the source and drain electrodes serve as contact holes, and connection holes which are formed on the passivation layer for insulating the source and drain electrodes from the lower electrode serve as via holes.

The lower electrode serves as an anode electrode, and the upper electrode serves as a cathode electrode.

Preferred embodiments of the present invention have been described, however, the description of the connection holes in the first and second embodiments may also be applied to the case of forming an opening of the planarization layer.

According to the present invention as mentioned above, surface corner portions of contact holes and via holes may be curved so that short-circuit failures may be reduced to enhance reliability and yield.

Although the present invention has been described with reference to certain exemplary embodiments thereof, it will be understood by those skilled in the art that a variety of modifications and variations may be made to the present invention without departing from the spirit or scope of the present invention defined in the appended claims, and their equivalents.

**Claims**

1. An organic light emitting display comprising:

a semiconductor layer formed on a substrate, and having source and drain regions;
a thin film transistor formed on the substrate, and having source and drain electrodes electrically connected to the semiconductor layer through a connection hole;
a lower electrode formed on an emission region of the substrate, and connected to one electrode of the source and drain electrodes through a connection hole;
an organic emission layer formed on the emission region of the lower electrode; and
a upper electrode formed on the organic emission layer,

wherein the connection hole has a curved surface corner portion.

2. The organic light emitting display as recited in claim 1, wherein: a corner of a bottom surface of the connection hole has a curvature; a center of the curvature is present on the bottom surface; a top surface of the connection hole has a point which is positioned on the same axis vertically extending from a surface including a center of a radius of curvature of the bottom surface, as a center of a radius of curvature; and the radius of curvature of the top surface of the connection hole is larger than that of the bottom surface.

3. The organic light emitting display as recited in claim 2, wherein all of the curvature of the top surface and the curvature of the bottom surface of the connection hole are formed at respective four corners.

4. The organic light emitting display as recited in claim 1, wherein:

    corners of the top and bottom surfaces of the connection hole are circular; a point positioned on the same axis vertically extending from a surface including the center of the radius of the top surface is the center of the radius of the bottom surface; and the radius of the top surface of the connection hole is larger than that of the bottom surface.

5. The organic light emitting display as recited in claim 1, wherein the connection hole formed on the insulating layer to insulate the source and drain regions from the source and drain electrodes serves as a contact hole.

6. The organic light emitting display as recited in claim 1, wherein the connection hole formed on the insulating layer to insulate the source and drain electrodes from the lower electrode serves as a via hole.

7. The organic light emitting display as recited in claim 1, wherein the lower electrode is an anode electrode and the upper electrode is a cathode electrode.

8. A method of fabricating an organic light emitting display, comprising:

    forming a semiconductor layer having source and drain regions on a substrate;
    forming a thin film transistor provided with source and drain electrodes electrically connected to the semiconductor layer through a connection hole on the substrate;
    forming a lower electrode connected to one electrode of the source and drain electrodes through a connection hole on an emission region of the substrate;
    forming an organic emission layer on the emission region of the lower electrode; and
    forming a upper electrode on the organic emission layer,

    wherein the connection hole has a curved surface corner portion.

9. The method as recited in claim 8, wherein: a corner of a bottom surface of the connection hole has a curvature; a center of the curvature is present on the bottom surface; a top surface of the connection hole has a point which is positioned on the same axis vertically extending from a surface including a center of a radius of curvature of the bottom surface, as a center of a radius of curvature; and the radius of curvature of the top surface of the connection hole is formed to be larger than that of the bottom surface.

10. The method as recited in claim 9, wherein all of the curvature of the top surface and the curvature of the bottom surface of the connection hole are formed at respective four corners.

11. The method as recited in claim 8, wherein: corners of the top and bottom surfaces of the connection hole are formed to be circular; and a point positioned on the same axis vertically extending from a surface including the center of the radius of the top surface is the center of the radius of the bottom surface; and the radius of the top surface of the connection hole is formed to be larger than that of the bottom surface.

# FIG. 1A
## (PRIOR ART)

FIG. 1B
(PRIOR ART)

# FIG. 1C
## (PRIOR ART)

# FIG. 1D
## (PRIOR ART)

161,162,171

II ———————————— II

# FIG. 1E
(PRIOR ART)

L1

L2

FIG. 1F
(PRIOR ART)

# FIG. 2A

FIG. 2B

# FIG. 2C

201a

201b

# FIG. 2D

161,162,171

ρ2

ρ1

IV ———— IV

261b

261a

# FIG. 2E

# FIG. 3A

# FIG. 3B

361a

r1

r2

361b

# FIG. 3C

# FIG. 3D

**European Patent Office**

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | US 6 569 717 B1 (MURADE MASAO) 27 May 2003 (2003-05-27) * column 22, line 47 - line 56 * ----- | 1-11 | H01L27/00 |
| Y | EP 1 333 497 A (SEIKO EPSON CORPORATION) 6 August 2003 (2003-08-06) * figure 3 * * page 3, lines 9-11 * ----- | 1-11 | |
| A | US 6 323 490 B1 (IKEDA MITSUSHI ET AL) 27 November 2001 (2001-11-27) * column 38, line 34 - line 41 * ----- | 1-4,6, 8-11 | |
| A | PATENT ABSTRACTS OF JAPAN vol. 2003, no. 05, 12 May 2003 (2003-05-12) -& JP 2003 022035 A (SHARP CORP), 24 January 2003 (2003-01-24) * abstract; figures * ----- | 1-4,6, 8-11 | |
| A | US 2002/036462 A1 (HIRANO TAKASHI) 28 March 2002 (2002-03-28) * figures 1,2 * * paragraph [0021] * ----- | 1-4,6, 8-11 | TECHNICAL FIELDS SEARCHED (Int.Cl.7)  H01L |
| A | EP 1 324 109 A (SANYO ELECTRIC CO., LTD) 2 July 2003 (2003-07-02) * figures 5a,6 * ----- | 1-11 | |

The present search report has been drawn up for all claims

| Place of search | Date of completion of the search | Examiner |
|---|---|---|
| The Hague | 8 March 2005 | De Laere, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 1 566 839 A1**

**ANNEX TO THE EUROPEAN SEARCH REPORT
ON EUROPEAN PATENT APPLICATION NO.**

EP 05 07 5208

08-03-2005

| Patent document cited in search report | | Publication date | Patent family member(s) | | Publication date |
|---|---|---|---|---|---|
| US 6569717 | B1 | 27-05-2003 | JP | 3399432 B2 | 21-04-2003 |
| | | | JP | 2000312006 A | 07-11-2000 |
| | | | JP | 2000312005 A | 07-11-2000 |
| EP 1333497 | A | 06-08-2003 | JP | 2003316296 A | 07-11-2003 |
| | | | CN | 1435804 A | 13-08-2003 |
| | | | EP | 1333497 A2 | 06-08-2003 |
| | | | JP | 2003323138 A | 14-11-2003 |
| | | | US | 2003214042 A1 | 20-11-2003 |
| US 6323490 | B1 | 27-11-2001 | JP | 3447947 B2 | 16-09-2003 |
| | | | JP | 11274446 A | 08-10-1999 |
| | | | JP | 11274524 A | 08-10-1999 |
| JP 2003022035 | A | 24-01-2003 | NONE | | |
| US 2002036462 | A1 | 28-03-2002 | JP | 2001356711 A | 26-12-2001 |
| | | | TW | 498701 B | 11-08-2002 |
| EP 1324109 | A | 02-07-2003 | JP | 2003202587 A | 18-07-2003 |
| | | | CN | 1432854 A | 30-07-2003 |
| | | | EP | 1324109 A1 | 02-07-2003 |
| | | | TW | 588205 B | 21-05-2004 |
| | | | US | 2003156239 A1 | 21-08-2003 |

EPO FORM P0459

For more details about this annex : see Official Journal of the European Patent Office, No. 12/82